# EUROPEAN PATENT APPLICATION

(11) **EP 0 943 679 A1**
(43) Date of publication of application: **22.09.1999**
(21) Application number: 98870053.0
(22) Date of filing: 13.03.1998
(51) Int. Cl.: C12N 9/22, C12N 15/55, C12Q 1/68, A61K 38/46, G01N 33/68

(54) **Novel RNase-like protein and its use**

(71) Applicant: INNOGENETICS N.V., 9052 Gent (BE)
(72) Inventor: The designation of the inventor has not yet been filed

(57) **Abstract**

The present invention relates to a new protein; and its coding nucleic acid sequence, derived from human keratinocytes. The latter protein is structurally similar to the pancreatic RNase family-members and is useful for diagnostic and/or therapeutic poses in the fields of oncology, virology and wound healing.

SEQUENCE LISTING

(1) GENERAL INFORMATION:

(i) APPLICANT:
(A) NAME: INNOGENETICS N.V.
(B) STREET: INDUSTRIEPARK ZWIJNAARDE 7, BOX 4
(C) CITY: GHENT
(E) COUNTRY: BELGIUM
(F) POSTAL CODE (ZIP): B9052
(G) TELEPHONE: 00 32 9 241 07 11
(H) TELEFAX: 00 32 9 241 07 99

(ii) TITLE OF INVENTION: NOVEL RNAse-LIKE FACTOR AND ITS USE.

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 1:

CARCARTTYC AYTTRGTCC 19

(2) INFORMATION FOR SEQ ID NO: 2:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 2:

CARCARTTYC AYCTNGTCC 19

(2) INFORMATION FOR SEQ ID NO: 3:

(i) SEQUENCE CHARACTERISTICS:
(A) LENGTH: 11 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 3:

Arg Lys Xaa Xaa Gln Gln Phe His Leu Val Pro
1               5                   10

(2) INFORMATION FOR SEQ ID NO: 4:

(i) SEQUENCE CHARACTERISTICS:
(A) LENGTH: 1140 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 4:

GAATTCGCGG CCGCGTCGAC CCAGCTCTTA AGGAGTTCAG GAGTGAGAAG AGGCCCTCAG 60

AGATCTGACA GCCTAGGAGT GCGTGGACAC CACCTCAGCC CACTGAGCAG GAGTCACAGC 120

ACGAAGACCA AGCGCAAAGC GACCCCTGCC CTCCATCCTG ACTGCTCCTC CTAAGAGAGA 180

TGGCACCGGC CAGAGCAGGA TTCTGCCCCC TTCTGCTGCT TCTGCTGCTG GGGCTGTGGG 240

TGGCAGAGAT CCCAGTCAGT GCCAAGCCCA AGGGCATGAC CTCATCACAG TGGTTTAAAA 300

TTCAGCACAT GCAGCCCAGC CCTCAAGCAT GCAACTCAGC CATGAAAAAC ATTAACAAGC 360

ACACAAAACG GTGCAAAGAC CTCAACACCT TCCTGCACGA GCCTTTCTCC AGTGTGGCCG 420

CCACCTGCCA GACCCCCAAA ATAGCCTGCA AGAATGGCGA TAAAAACTGC CACCAGAGCC 480

ACGGGCCCGT GTCCCTGACC ATGTGTAAGC TCACCTCAGG GAAGTATCCG AACTGCAGGT 540

ACAAAGAGAA GCGACAGAAC AAGTCTTACG TAGTGGCCTG TAAGCCTCCC CAGAAAAAGG 600

ACTCTCAGCA ATTCCACCTG GTTCCTGTAC ACTTGGACAG AGTCCTTTAG GTTTCCAGAC 660

TGGCTTGCTC TTTGGCTGAC CTTCAATTCC CTCTCCAGGA CTCCGCACCA CTCCCCTACA 720

CCCAGAGCAT TCTCTTCCCC TCATCTCTTG GGGCTGTTCC TGGTTCAGCC TCTGCTGGGA 780

GGCTGAAGCT GACACTCTGG TGAGCTGAGC TCTAGAAGGA TGGCTTTTCA TCTTTTTGTT 840

GCTGTTTTCC CAGATGCTTA TCCCCAAGAA ACAGCAAGCT CAGGTCTGTG GGTTCCCTGG 900

TCTATGCCAT TGCACATGTC TCCCCTGCCC CCTGGCATTA GGGCAGCATG ACAAGGAGAG 960

GAAATAAATG GAAAGGGGGC ATATGGGATT TGTGGACACA GCTGTTTCTG TTCCTGAACT 1020

AGAAGTCTTC CCCAGCTCTG ACGTGGCAGT GAGGTGACCT GAAGGAAAGA AAAATATAAA 1080

TAAATACCAC TTCATATTTG TATAGAAAAA AAAAAAAAAA GTCGACGCGG CCGCGAATTC 1140

(2) INFORMATION FOR SEQ ID NO: 5:

(i) SEQUENCE CHARACTERISTICS:
(A) LENGTH: 156 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 5:

Met Ala Pro Ala Arg Ala Gly Phe Cys Pro Leu Leu Leu Leu Leu Leu
1               5                   10                  15

Leu Gly Leu Trp Val Ala Glu Ile Pro Val Ser Ala Lys Pro Lys Gly
            20                  25                  30

Met Thr Ser Ser Gln Trp Phe Lys Ile Gln His Met Gln Pro Ser Pro
        35                  40                  45

Gln Ala Cys Asn Ser Ala Met Lys Asn Ile Asn Lys His Thr Lys Arg
    50                  55                  60

Cys Lys Asp Leu Asn Thr Phe Leu His Glu Pro Phe Ser Ser Val Ala

65                  70                  75                  80

Ala Thr Cys Gln Thr Pro Lys Ile Ala Cys Lys Asn Gly Asp Lys Asn
                85                  90                  95

Cys His Gln Ser His Gly Pro Val Ser Leu Thr Met Cys Lys Leu Thr
            100                 105                 110

Ser Gly Lys Tyr Pro Asn Cys Arg Tyr Lys Glu Lys Arg Gln Asn Lys
        115                 120                 125

Ser Tyr Val Val Ala Cys Lys Pro Pro Gln Lys Lys Asp Ser Gln Gln
    130                 135                 140

Phe His Leu Val Pro Val His Leu Asp Arg Val Leu
145                 150                 155

(2) INFORMATION FOR SEQ ID NO: 6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 6:

TAGAATCCTC CTAAGAGAGA TGGCACC                                    27

(2) INFORMATION FOR SEQ ID NO: 7:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 7:

GACTCGAGCA GTCTGGAAAC CTAAAGGAC                                  29

(2) INFORMATION FOR SEQ ID NO: 8:

(i) SEQUENCE CHARACTERISTICS:
(A) LENGTH: 56 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 8:

CACTCGAGCT AGTGGTGGTG GTGGTGGTGG GAACCAAGGA CTCTGTCCAA GTGTAC 56

(2) INFORMATION FOR SEQ ID NO: 9:

(i) SEQUENCE CHARACTERISTICS:
(A) LENGTH: 164 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 9:

Met Ala Pro Ala Arg Ala Gly Phe Cys Pro Leu Leu Leu Leu Leu Leu
1               5                   10                  15

Leu Gly Leu Trp Val Ala Glu Ile Pro Val Ser Ala Lys Pro Lys Gly
            20                  25                  30

Met Thr Ser Ser Gln Trp Phe Lys Ile Gln His Met Gln Pro Ser Pro
        35                  40                  45

Gln Ala Cys Asn Ser Ala Met Lys Asn Ile Asn Lys His Thr Lys Arg
    50                  55                  60

Cys Lys Asp Leu Asn Thr Phe Leu His Glu Pro Phe Ser Ser Val Ala
65                  70                  75                  80

Ala Thr Cys Gln Thr Pro Lys Ile Ala Cys Lys Asn Gly Asp Lys Asn
                85                  90                  95

Cys His Gln Ser His Gly Pro Val Ser Leu Thr Met Cys Lys Leu Thr

100                 105                 110

Ser Gly Lys Tyr Pro Asn Cys Arg Tyr Lys Glu Lys Arg Gln Asn Lys
        115                 120                 125

Ser Tyr Val Val Ala Cys Lys Pro Pro Gln Lys Lys Asp Ser Gln Gln
    130                 135                 140

Phe His Leu Val Pro Val His Leu Asp Arg Val Leu Gly Ser His His
145                 150                 155                 160

His His His His

(2) INFORMATION FOR SEQ ID NO: 10:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 10:

CAGTTTAAAC CCAAGGGCAT GACCTCATC                                    29

(2) INFORMATION FOR SEQ ID NO: 11:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 11:

AGTCTAGAAA CCTAAAGGAC TCTG                                         24

(2) INFORMATION FOR SEQ ID NO: 12:

(i) SEQUENCE CHARACTERISTICS:

(A) LENGTH: 136 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 12:

Met Ala His His His His His His Lys Pro Lys Gly Met Thr Ser Ser
1               5                   10                  15

Gln Trp Phe Lys Ile Gln His Met Gln Pro Ser Pro Gln Ala Cys Asn
            20                  25                  30

Ser Ala Met Lys Asn Ile Asn Lys His Thr Lys Arg Cys Lys Asp Leu
        35                  40                  45

Asn Thr Phe Leu His Glu Pro Phe Ser Ser Val Ala Ala Thr Cys Gln
    50                  55                  60

Thr Pro Lys Ile Ala Cys Lys Asn Gly Asp Lys Asn Cys His Gln Ser
65                  70                  75                  80

His Gly Pro Val Ser Leu Thr Met Cys Lys Leu Thr Ser Gly Lys Tyr
                85                  90                  95

Pro Asn Cys Arg Tyr Lys Glu Lys Arg Gln Asn Lys Ser Tyr Val Val
            100                 105                 110

Ala Cys Lys Pro Pro Gln Lys Lys Asp Ser Gln Gln Phe His Leu Val
        115                 120                 125

Pro Val His Leu Asp Arg Val Leu
    130                 135

## Description

### FIELD OF THE INVENTION

The present invention relates to a new protein, and its coding nucleic acid sequence, derived from human keratinocytes. The latter protein is structurally similar to the pancreatic RNase family-members and is useful for diagnostic and/or therapeutic purposes in the fields of oncology, virology and wound healing.

### BACKGROUND OF THE INVENTION

RNases are amongst the oldest enzymes Although traditionally considered as mere housekeeping enzymes, they are more and more being implicated in a variety of physiological and biochemical processes. The expression of many RNases is tightly regulated and their distribution may be very tissue or developmental stag-specific. The intracellular RNase content of cells my depend both upon endogenous production and on uptake of enzymes of pancreatic origin. RNase activity may be controlled by endogenous expression level, degree of exogenous uptake and by the presence of endogeuous RNase inhibitors or activators. Moreover, the target specificity may vary considerably from one RNase to another (Schein, 1997).

A common property of several secreted RNase is the fact that they are cytotoxic towards a variety of cells. For instance, RNase 2, also called eosinophil-derived neurotoxin (EDN) is cytotoxic for neuronal cells. When this molecule is injected intraventricularly, it causes the so-called Gordon syndrome, a condition characterized by ataxia, muscular rigidity, paralysis and tremor, that may eventually lead to death of Purkinje neurons (Newton et al., 1994a). A similar factor is eosinophil-derived cationic protein (ECP, or ribonuclease 3), which also has bactericidal properties (Rosenberg et al., 1989). Onconase is a Xenopus RNase which is highly cytotoxic for certain tumor cells and is also capable of inducing the Gordon phenomenon when injected into the cerebrospinal fluid (Newton et al. 1994a).

Another member of the pancreatic RNase family is angiogenin, a 14 kDa peptide originally purified from the culture supernatants of human adenocarcinoma cells (Fett et al., 1985). The protein is a potent inducer of neovascularization in a number of standard *in vivo* assays for angiogenesis such as the chicken chorioallantois membrane (CAM) and rabbit cornea assays. Angiogenin has also been reported to induce endothelial tube formation and collagen gel invasion in an *in vitro* angiogenesis assay (Jimi et al., 1995). Although belonging to the pancreatic RNase family (33% homology with bovine RNase A), angiogenin itself has a relatively weak RNase activity, with a specific activity which is 10⁴-10⁵-fold lower than that of RNaseA (with tRNA as a substrate; Lee and Vallee, 1989). Several studies have indicated that the angiogenic activity of the factor is dependent on the presence of ribonucleolytic activity. This has been concluded on basis of sit-directed mutagenesis experiments, in which mutations leading to an increased or decreased ribonucleolytic activity led to a proportional increase or decrease of angiogenic potential (Shapiro and Vallee, 1989). Besides its RNase and angiogenic properties, angiogenin has also been found to display cytotoxic activity when injected into Xenopus oocytes, an effect which has been shown to be mediated by tRNA breakdown (Saxena et al., 1992).

The cytotoxic properties of certain RNases may lead to the development of novel therapeutics. For insance, when administered together with vincristine, the tumoricidal effect of onconase leads to a significant increase of the mean surival time of mice carrying vincristine-resistant tumors (Newton et al., 1996a). Onconase is currently being clinically evaluated for the treatment of breast cancer and mesothelioma (phase II) and for pancreatic cancer (ph III) by the US-based company Alfacell Both EDN and human pancreatic RNase have been fused to a single chain antibody against the transferrin receptor and the resulting constructs were cytotoxic towards several human tumor cell lines expressing the transferrin receptor but not towards cells lacking the transferrin receptor (Zewe et al., 1997, Newton et al., 1994b). Similar findings were made with pancreatic RNase linked to EGF (Jinno et al, 1996) and with angiogenin linked to transferrin (Newton et al., 1996b).

In a completely different therapeutic domain, onconase and bovine seminal RNase have been shown to inhibit the replication of HIV in infected H9 leukemia cells at concentrations not toxic for the cells (Youle et al., 1994). Northern blot analysis suggests that onconase, once entered into the cell, degrades the viral RNA but does not degrade cellular rRNA or mRNA (Saxena et al., 1996). Recently, an 18 kDa human urinary RNase was reported to inhibit Kaposi sarcoma cells by induction of apoptosis (Griffiths et al., 1997).

In the field of tissue repair, applications may be found for angiogenin, which may promote healing in situations were decreased vascularization lays at the basis of the pathology. For instance, King and Vallee (1991) have shown that implantation of angiogenin in experimentally injured menisci in a rabbit model significantly induced vascularisation in comparison with controls.

With respect to possible diagnostic applications, it has been observed that certain RNases are upregulated in a number of pathological conditions. For instance, RNase L levels are increased in colorectal carcinoma, CML and chronic fatigue syndrome patients. EDN is increased in vernal conjunctivitis and pancreatic RNase in pancreatic necrosis (Schein, 1997).

In view of the data above, it is clear that the members of the pancreatic RNase family are important molecules with a variety of functions. Several of them have therapeutic potential and at least one of them is already being tested clinically as an anti-cancer drug.

### AIM OF THE INVENTION

The present invention relates to the discovery and isolation of a new member of the pancreatic RNase family, named keratinocyte-derived RNase-like factor (KRF), which is useful for diagnostic and/or therapeutic purposes in the fields of oncology, virology and wound healing. Therefore, the present invention aims at providing an isolated nucleic acid sequence encoding a polypeptide having the amino acid sequence of SEQ ID 5 (see further), or a derivative thereof, having RNase, angiogenic, antiviral, cytotoxic or cytostatic activity, selected from the group consisting of:
a. the DNA sequence given by SEQ ID 4 (see further) or the complementary strand thereof,
b. DNA sequences which hybridize under stringent conditions to the protein coding regions of the DNA sequence defined in (a) or fragments thereof, and
c. DNA sequences which, for the degeneracy of the genetic code, would hybridize to the DNA sequences defined in (a) and (b).

The present invention also aims at providing a vector comprising a nucleic acid as described above and a host cell comprising said vector.

The present invention further aims at providing a polypeptide having part or all of the amino acid sequence of SEQ ID 5 or any derivative thereof having RNase, angiogenic, antiviral, cytotoxic or cytostatic activity.

Furthermore, the present invention aims at providing a pharmaceutical composition comprising a polypeptide as described above, or any functionally equivalent variant or fragment thereof having RNase, angiogenic, antiviral, cytotoxic or cytostatic activity, and a pharmaceutically acceptable carrier.

Consequently, the present invention aims at providing a method for promoting angiogenesis in a mammal comprising the step of administering to said a pharmaceutical composition as described above.

The present invention also aims at providing a method for promoting wound healing in a mammal comprising the step of administering to said mammal a pharmaceutical composition as described above.

The present invention further aims at providing a method for lowering the amount of intracellular or extracellular RNA in a mammal comprising the step of administering to said mammal a pharmaceutical composition as described above.

Moreover, the present invention aims at providing a pharmaceutical composition as described above for use as medicament to treat a microbial infection.

More specifically, the present invention aims at providing a pharmaceutical composition as described above wherein said microbial infection is a viral infection.

Even more specifically, the present invention aims at providing a pharmaceutical composition as described above wherein said viral infection is caused by a RNA virus.

Furthermore ,the present invention aims at providing a pharmaceutical composition as described above wherein said RNA virus belongs to a RNA virus family chosen from the group consisting of Reoviridae, Coronaviridae, Flaviviridae, Picornaviridae, Togaviridae, Paramyxoviridae, Rhabdoviridae, Bunyaviridae, Orthomyxoviridae or Retroviridae.

The present invention further aims at providing a pharmaceutical composition as described above for use as medicament to treat malignancies and/or hyperproliferative disorders.

The present invention also aims at providing a method for detecting a nucleic acid sequence as described above comprising:
- contacting said nucleic acid with a probe
- determining the complex formed between said nucleic acid and said probe.

In addition, the present invention aims at providing a method for detecting a polypeptide as described above comprising:
- contacting said polypeptide with a ligand binding to said polypeptide
- determining the complex formed between said polypeptide and said ligand.

Finally, the present invention aims at providing a ligand binding to a polypeptide as described above.

All the aims of the present invention are considered to have been met by the embodiments as set out below.

### BRIEF DESCRIPTION OF TABLES AND FIGURES

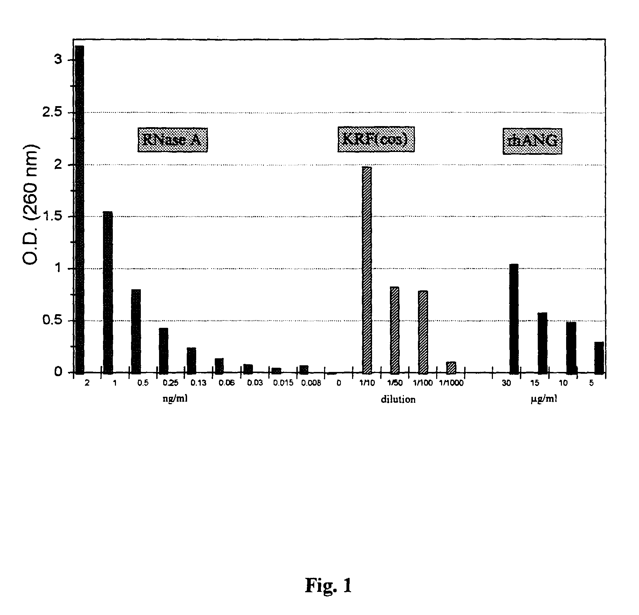

- **Fig. 1:**: **RNase activity of cos-expressed recombinant KRF, bovine pancreatic RNase A and human recombinant Angiogenin.**
All samples were evaluated using tRNA as a substrate. The amount of non-acid-precipitable nucleic acid generated was determined as a measure of RNase activity. Conditioned medium from the cos cells was tested at dilutions 1/10-1/1000. *E. coli*-expressed recombinant Angiogenin (R&D systems) was tested at 5-30 µg/ml. All OD values shown were obtained after subtraction of blanc values (incubation in absence of RNase). In case of cos-expressed KRF, blanc values for each point were obtained by assaying a sample of mock-transfected cos cell conditioned medium at the same dilution to correct for the low presence of RNase activity produced intrinsically by the cells (in general, this RNase activity was about 5% of that present in the KRF-tranfected cells).

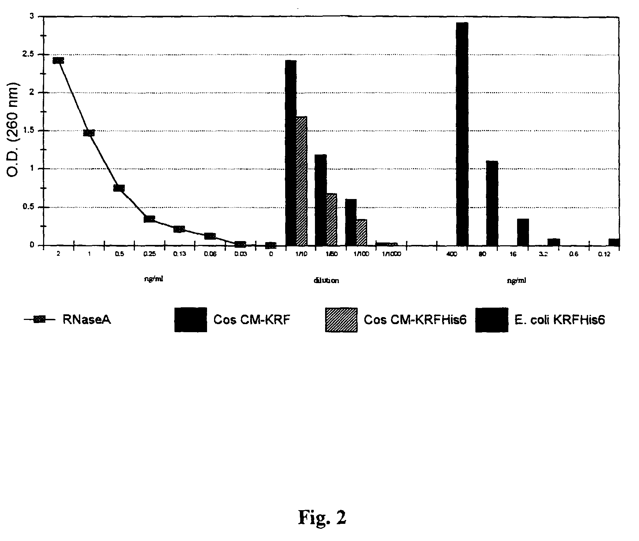

- **Fig. 2:**: **RNase activity of non-purified conditioned medium of cos-cells transfected with a mature KRF and with a KRF-His6 expression construct and of purified E. coli-expressed His6-KRF.**
All samples were evaluated using tRNA as a substrate. The amount of non-acid-precipitable nucleic acid generated was determined as a measure of RNase activity. Conditioned medium from the cos cells was tested at dilutions 1/10-1/1000. Purified *E. coli* His6-KRF was tested at 0.12-400 ng/ml. All OD values shown were obtained after subtraction of blanc values (incubation in absence of RNase). For comparison, bovine pancreatic RNase A was also included.

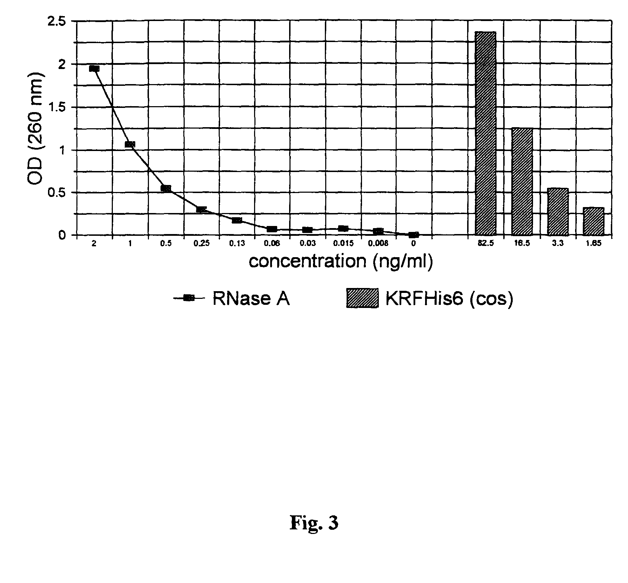

- **Fig. 3:**: **RNase activity of recombinant KRF-His6, purified from cos cell conditioned medium.**
All samples were evaluated using tRNA as a substrate. The amount of non-acid-precipitable nucleic acid generated was determined as a measure of RNase activity. Purified cos KRF-His6 was tested at 1.65-82.5 ng/ml. All OD values shown were obtained after subtraction of blanc values (incubation in absence of RNase). For comparison, bovine pancreatic RNase A was also included.

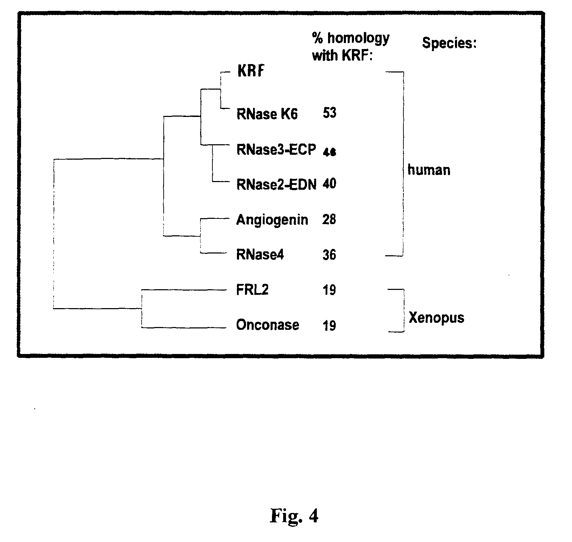

- **Fig. 4:**: **Homology tree of different members of the pancreatic RNase family.**
Protein sequences of the different indicated members of the pancreatic RNase family were aligned using the program "clustal" (PCGene). Using the same program, a homology tree was constructed, on which the percentage of homology with KRF is indicated.

### DETAILED DESCRIPTION OF THE INVENTION

The invention described herein draws on previously published work and pending patent applications. By way of example, such work consists of scientific papers, patents or pending patent applications. All these publications and applications, cited previously or below are hereby incorporated by reference.

The present invention is based on the finding that a polypeptide isolated from keratinocytes has RNase, angiogenic, antiviral, cytotoxic or cytostatic activity. Therefore, the present invention aims at providing a isolated nucleic acid sequence encoding a polypeptide haing the amino acid sequence of SEQ ID 5 (see above), or a derivative thereof, having RNase, angiogenic, antiviral, cytotoxic or cytostatic activity, selected from the group consisting of
a. the DNA sequence given by SEQ ID 4 (see above) or the complementary strand thereof,
b. DNA sequences which hybridize under stringent conditions to the protein coding regions of the DNA sequence defined in (a) or fragments thereof, and
c. DNA sequences which, for the degeneracy of the genetic code, would hybridize to the DNA sequences defined in (a) and (b).

The term "polypeptide" refers to a polymer of amino acids (aa) or fragments thereof having RNase, angiogenic, antiviral, cytotoxic or cytostatic activity. The term "isolated" refers to material which is free from components which normally accompany it as found in its naturally occuring environment. However, it should be clear that the isolated polypeptide of the present invention may comprise heterologous cell components, a ligand binding moiety (such as an antibody or any antibody fragment known in the art as described in EP 97870092.0 to Lorré et al, or, any receptor known in the art), a label and the like. Furthermore, the term "derivative" of said polypeptide refers to any variant or fragment of the polypeptide represented by SEQ ID 5 which retains RNase, angiogenic, antiviral, cytotoxic or cytostatic activity. The latter term includes post-translational modifications of said polypeptides such as glycosylation, acetylation, phosphorylation, modifications with fatty acids and the like. Also included within the definition are, for example, polypeptides containing one or more analogues of an aa (including unnatural aa's), polypeptides with substituted linkages, mutated versions or natural sequence variations of the polypeptides, polypeptides containing disulfide bounds between cysteine residues, biotinylated polypeptides as well as other modifications known in the art. When percentage of sequence identity is used in reference to polypeptides, it is recognized that residue positions which are not identical often differ by conservative aa substitutions, where aa residues are substituted for other aa residues with similar chemical properties (for example charge or hydrophobicity) and therefore do not change the functional properties of the polypeptide. Where sequences differ in conservative substitutions, the percent sequence identity may be adjusted upwards to correct for the conservative nature of the substitution. Means for making this adjustment are well-known to those of skill in the art. Typically this involves scoring a conservative substitution as a partial rather than a full mismatch, thereby increasing the percentage sequence identity. Thus, for example (and as described in WO 97/31116 to Rybak et al.), where an identical aa is given a score of 1 and a non-conservative substitution is given a score of zero, a conservative substitution is given a score between zero and 1. In this regard, it should be clear that polypeptides, or parts thereof, comprising an aa sequence with a least 55%, preferably 75%, more preferably 85% or most preferably 90% sequence identity with the aa sequence given by SEQ ID 5, or a part thereof, fall within the scope of the present invention. It should also be clear that polypeptides which are immunologically reactive with antibodies raised against the polypeptide having the aa sequence given by SEQ ID 5 fall within the scope of the present invention.

The polypeptides of the present invention are expressed in human keratinocytes. However, it should be clear that the polypeptides of the present invention can be isolated or extracted from any cell or cell line by any method known in the art such as the ones described in WO/9709349 to Abrignani and can be characterized and sequenced by any method known in the art. The latter polypeptides can also be produced by means of recombinant DNA techniques as described by Maniatis et al. (1982) and in the *Examples* section of the present application or by any other method known in the art such as classical chemical synthesis as described by Houbenweyl (1974) and Atherton & Shepard (1989).

The term "RNase activity" (or ribonuclease activity) refers to any activity which results into the hydrolysis of RNA The RNA molecules to be hydrolyzed can be located intracellularly or extracellularly and can be derived from any organism

The term "angiogenic activity" refers to any activity which stimulates the formation of new blood vessels, either *de novo* or by sprouting from existing blood vessels.

The term "antiviral activity" refers to any activity which inhibits the replication or formation of viruses or which inhibits the infectivity of viruses or which inhibits the pathogenicity of viruses.

The term "cytotoxic activity" refers to any activity which is poisonous for cells and/or may cause cell death. The latter activity my disturb fundamental cell mechanisms involving growth, differentiation, metabolism, intercellular interactions, cell-matrix interactions or other cell functions. The latter cells can be of any origin, including prokaryotic cells (such as bacteria), lower eukaryotic cells (such as yeasts) or higher eukaryotic cells (such as mammalian cells).

The term "cytostatic activity" refers to any activity which inhibits proliferation or replication of cells. The latter cells can be of any origin, including prokaryotic cells (such as bacteria), lower eukaryotic cells (such as yeasts) or higher eukaryotic cells (such as mammalian cells).

The term "nucleic acid" refers to a deoxyribonucleotide or ribonucleotide polymer in either single- or double stranded form which may encompass known analogues of natural nucleotides that hybridize to nucleic acids in a manner similar to naturally occuring nucleotides. The term specifically refers to the DNA sequence given by SEQ ID 4 or the complementary strand thereof Also within the scope of the present invention are nucleic acids which hybridize under stringent conditions to the protein coding regions of the DNA sequence given by SEQ ID 4 or fragments thereof. Stringent conditions are sequence dependent and are different under different environmental parameters. Generally, stringent conditions are selected to be about 5°C to 20°C lower than the termal melting point (Tₘ) for the specific sequence at a defined ionic strenght and pH. The Tₘ is the temperature (under defined ionic strenght and pH) at which 50% of the target sequence hybridizes to a perfectly matched probe. However, nucleic acids which do not hybridize to each other under stringent conditions can still encode a polypeptide of the present invention as described above. This occurs, for example, when a copy of a nucleic acid is created using the maximum codon degeneracy permitted by the genetic code. Therefore, DNA sequences which, for the degeneracy of the genetic code, *would* hybridize to the DNA sequences as defined above, fall within the scope of the present invention. Also nucleic acids which encode a polypeptide which is immunologically reactive with antibodies raised against the polypeptide encoded by the DNA sequence given by SEQ ID 4 fall within the scope of the present invention.

The term "vector" refers to a vector sequence of the type plasmid, cosmid, phage or viral DNA wherein a nucleic acid which encodes KRF, or any derivative thereof, is inserted and which contains the necessary elements to promote the transcription and translation of the latter polypeptide(s) by a host cell. The term "host cell" relates to any prokaryotic cellular host chosen from bacteria such as *Escherichia coli* and *Bacillus* species, or, any eukaryotic host cell such as COS cells or *Saccharomyces cerevisae* which is transformed by a recombinant vector defined above.

The terms "a pharmaceutical composition" relates to a composition or medicament (both terms can be used interchangeably) comprising a polypeptide of the present invention and a pharmaceutically acceptable carrier or excipient (both terms can be used interchangeably) to promote angiogenesis or wound healing, and/or, to treat microbial infections, malignancies and/or hyperproliferative disorders. Suitable carriers or excipients known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrier that does not itself induce the production of antibodies harmful to the individual receiving the composition such as proteins, polysaccharides, polylactic acids, polyglycolic acids, polymeric amino acids and amino acid copolymers. The "pharmaceutical composition" or "medicament" may be administered by any suitable method within the knowledge of the skilled man. The preferred route of administration is parenterally, or, by direct topical application to the wound site. In parental administration, the medicament of this invention will be formulated in a unit dosage injectable form such as a solution, suspension or emulsion, in association with the pharmaceutically acceptable excipients as defined above. However, the dosage and mode of administration will depend on the individual. Generally, the medicament is administered so that the polypeptide of the present invention is given at a dose between 1 µg/kg and 10 mg/kg, more preferably between 10 µg/kg and 5 mg/kg, most preferably between 0.1 and 2 mg/kg. Preferably, it is given as a bolus dose. Continuous infusion may also be used. If so, the medicament may be infused at a dose between 5 and 20 µg/kg/minute, more preferably between 7 and 15 µg/kg/minute. With regard to direct topical administration, the pharmaceutical composition of the present invention preferably comprises the polypeptides of the present invention in a formulation suitable for application onto surface wounds. Such a formulation can be applied directly to a surface wound either as a dry powder or in the form of a gel, a creme, an ointment, a suspension, a solution, or a biocompatible, synthetic or natural solid matrix, any of which can be prepared in a conventional manner, and, if desired, in addition of conventional, pharmaceutically acceptable excipients and additives. Normally, the polypeptides of the present invention are incorporated in such a pharmaceutical composition in a concentration which depends on the type of surface wound to be healed and the circumstances under which the composition is to be used. It should also be clear that the pharmaceutical composition of the present invention may comprise a functionally equivalent variant or fragment of the sequence given by SEQ ID 5. The latter terms refer to a molecule which contains the full protein sequence of KRF or part of the protein sequence of KRF, to which certain modifications have been applied, and which retains all or part of the biological properties of KRF. Such modifications include but are not limited to the addition of polysaccharide chains, the addition of certain chemical groups, the addition of lipid moieties, the fusion with other peptide or protein sequences and the formation of intramolecular cross-links.

The present invention further relates to a method for lowering the amount of intracellular or extracellular RNA in a mammal by administering the above-described pharmaceutical composition. The term "mammal" specifically refers to humans but does not exclude other mammals such as mice, rodents, non-human primates and the like. It should also be clear that the intra- or extracellular RNA to be hydrolyzed by the pharmaceutical composition of the present invention refers to microbial RNA (i.e viral, bacterial, protozoal and/or fungal RNA), mammalian RNA or RNA of any other origin.

The terms "a microbial infection" refer to an infection caused by viruses, bacteria, protozoa and/or fungi.

The terms "an RNA virus" refer to a virus of which the genome consists of single-stranded or double-stranded RNA. These viruses include the double stranded RNA viruses (such as the Reoviridae), the positive strand RNA viruses (such as the Coronaviridae, the Flaviviridae, the Picornaviridae and the Togaviridae), the negative strand RNA viruses (such as the Paramyxoviruses, the Rhabdoviridae, the Bunyaviridae and the Orthomyxoviridae) and the RNA reverse transcribing viruses (such as the Retroviridae including HIV).

The term "malignancy", as applied to tumours, refers to the fact that a primary tumour has the capacity to metastasise and implies loss of both growth- and positional control. The term "tumour" refers to any abnormal swelling and, more specifically, refers to a mass of neoplastic cells. The term "neoplasia" literally means "new growth" and usually refers to abnormal new growth (or tumour) which may be benign or malignant. Unlike hyperplasia (see further regarding hyperproliferative disorders), neoplastic proliferation persists even in the absence of the original stimulus.

The term "hyperproliferative disorder" refers to any disease associated with an increased proliferation rate of certain cells (as compared to the proliferation rate in healthy individuals).

The terms "a method for detecting a nucleic acid of the present invention with a probe" ad "a method for detecting a polypeptide of the present invention with a ligand" refer to any method known in the art as described in detail in WO 96/13590 to Maertens & Stuyver and Coligan et al. 1992.

The term "a ligand" refers to any molecule able to bind the polypeptides of the present invention. The latter term specifically refers to polyclonal and/or monoclonal antibodies specifically raised (by any method know in the art) against the polypeptides of the present invention and also encompasses any antibody-like, and other, constructs as described in detail in EP 97870092.0 to Lorré et al.

The present invention will now be illustrated by reference to the following examples which set forth particularly advantageous embodiments. However, it should be noted that these embodiments are illustrative and can not be construed as to restrict the invention in any way.

### EXAMPLES

### Example 1: Isolation of Keratinocyte-derived RNase-bike Factor (KRF) cDNA clones from a keratinocyte library

### Keratinocyte cDNA library construction

Human primary keratinocytes were isolated from skin biopsies of healthy donors and cultured in the presence of mitomycin-C-treated Swiss 3T3 fibroblast feeder cells according to Green et al (1979). Cells were cultured till passage 2 and allowed to stratify into multilayered epithelial sheets after reaching confluence. RNA was isolated from these cells using the method of Chomzcynski and Sacchi (1987) and used for the preparation of polyA(+) RNA. This polyadenylated RNA was then used for the construction of a cDNA library in the lambda gt11 vector.

### Screening of the keratinocyte library

The keratinocyte cDNA library was screened by hybridization using a mixture of two oligonucleotide probes (probe 6994: SEQ ID 1 and probe 6995: SEQ ID 2):
- SEQ ID 1:: probe 6994: 5' CARCARTTYCAYTTRGTICC 3'
- SEQ ID 2:: probe 6995: 5' CARCATTYCAYCTNGTICC 3'
The sequence of these oligonucleotide probes was derived from a peptide sequence ((R, K)XXQQFHLVP, SEQ ID 3) which was originally identified during the course of a research program on the purification of keratinocyte-derived autocrine growth factors. Screening a total of 500,000 to 1,000,000 pfu yielded 14 positive signals. Six of these positive clones were further analysed by PCR-subcloning using flanking lambda primers and gel electrophoresis. One of these clones (HB3608, derived from lambda clone λgt10.6994+95.4) was found to encode a novel protein with sequence homology with human angiogenin and to members of the pancreatic RNase family (Fig. 4). Based on this homology, the novel protein has been named Keratinocyte-derived RNase-like Factor (KRF).

### Subcloning and sequencing

To eliminate the possibility of PCR-derived cloning artefacts, the insert of lambda clone λgt10.6994+95.4 was excised by cutting with the restriction enzyme EcoRI and recloned into the EcoRI site of plasmid vector pBLSK(+). Cue of the resulting subclone was called HB3661 and the sequence of its insert found to correspond completely to that of clone HB3608. The complete cDNA sequence of this insert is given in SEQ ID 4:

10         20         30         40         50         60
|          |          |          |          |          |
1 GAATTCGCGG CCGCGTCGAC CCAGCTCTTA AGGAGTTCAG GAGTGAGAAG AGGCCCTCAG

61 AGATCTGACA GCCTAGGAGT GCGTGGACAC CACCTCAGCC CACTGAGCAG GAGTCACAGC

121 ACGAAGACCA AGCGCAAAGC GACCCCTGCC CTCCATCCTG ACTGCTCCTC CTAAGAGAGA

181 TGGCACCGGC CAGAGCAGGA TTCTGCCCCC TTCTGCTGCT TCTGCTGCTG GGGCTGTGGG

241 TGGCAGAGAT CCCAGTCAGT GCCAAGCCCA AGGGCATGAC CTCATCACAG TGGTTTAAAA

301 TTCAGCACAT GCAGCCCAGC CCTCAAGCAT GCAACTCAGC CATGAAAAAC ATTAACAAGC

361 ACACAAAACG GTGCAAAGAC CTCAACACCT TCCTGCACGA GCCTTTCTCC AGTGTGGCCG

421 CCACCTGCCA GACCCCCAAA ATAGCCTGCA AGAATGGCGA TAAAAACTGC CACCAGAGCC

481 ACGGGCCCGT GTCCCTGACC ATGTGTAAGC TCACCTCAGG GAAGTATCCG AACTGCAGGT

541 ACAAAGAGAA GCGACAGAAC AAGTCTTACG TAGTGGCCTG TAAGCCTCCC CAGAAAAAGG

601 ACTCTCAGCA ATTCCACCTG GTTCCTGTAC ACTTGGACAG AGTCCTTTAG GTTTCCAGAC

661 TGGCTTGCTC TTTGGCTGAC CTTCAATTCC CTCTCCAGGA CTCCGCACCA CTCCCCTACA

721 CCCAGAGCAT TCTCTTCCCC TCATCTCTTG GGGCTGTTCC TGGTTCAGCC TCTGCTGGGA

781 GGCTGAAGCT GACACTCTGG TGAGCTGAGC TCTAGAAGGA TGGCTTTTCA TCTTTTTGTT

841 GCTGTTTTCC CAGATGCTTA TCCCCAAGAA ACAGCAAGCT CAGGTCTGTG GGTTCCCTGG

901 TCTATGCCAT TGCACATGTC TCCCCTGCCC CCTGGCATTA GGGCAGCATG ACAAGGAGAG

961 GAAATAAATG GAAAGGGGGC ATATGGGATT TGTGGACACA GCTGTTTCTG TTCCTGAACT

1021 AGAAGTCTTC CCCAGCTCTG ACGTGGCAGT GAGGTGACCT GAAGGAAAGA AAAATATAAA

1081 TAAATACCAC TTCATATTTG TATAGAAAAA AAAAAAAAAA GTCGACGCGG CCGCGAATTC

Total number of bases is: 1140.
DNA sequence composition:    295 A;   329 C;   273 G;   243 T; 0 OTHER;

The corresponding amino acid sequence of human KRF is given by SEQ ID 5:

5 10 15 20 25 30
| | | | | |
1 M A P A R A G F C P L L L L L L G L W V A E I P V S A K P
31 K G M T S S Q W F K I Q H M Q P S P Q A C N S A M K N I N K
61 H T K R C K D L N T F L H E P F S S V A A T C Q T P K I A C

91 K N G D K N C H Q S H G P V S L T M C K L T S G K Y P N C R
121 Y K E K R Q N K S Y V V A C K P P Q K K D S Q Q F H L V P V
151 H L D R V L
Number of residues : 156.
Molecular weight (MW) : 17471 ; Checking number (CN) : 130715

On basis of the homology between this protein sequence and that of a number of RNases, the protein encoded by SEQ ID 5 has been named Keratinocyte-derived RNase-like Factor (KRF).

### Example 2: Recombinant expression of KRF

### Eukaryotic expression in cos cells

### Expression construct encoding the native protein

The protein-encoding region from the insert of clone HB3661 was subcloned into the cos expression vector pSVDE51, using a PCR-based approach using primers N° 7769 (SEQ ID 6) and N° 7770 (SEQ ID 7). This yielded subclone pSVDE51KRF, encoding the entire KRF protein, and including 14 and 12 nucleotides of 5'- and 3'-untranslated sequence, respectively. In this vector, expression of KRF is under control of the SV40 early promoter.

SEQ ID 6:
5' TAGAATCCTCCTAAGAGAGATGGCACC 3'
EcoRI Met
SEQ ID 7:
5' GACTCGAGCAGTCTGGAAACCTAAAGGAC 3'
XhoI Stop

### Expression construct encoding a C-terminal (His)6 fusion protein

Apart from the native protein, a second construct was made encoding a (His)6-tailed protein. Oligo-histidine-containing proteins are generally easier to purify because they display high affinity for metal ions, allowing immobilized metal ion affinity chromatography (IMAC). Therefore, the protein-encoding region of clone HB3661 was subcloned into vector pSVDE51 by PCR using primer N° 7769 (SEQ ID 6) and primer N° 7771 (SEQ ID 8). This yielded clone pSVDE51ALFH6, encoding KRF fused to the sequence GSHHHHHH at its C-terminal end (SEQ ID 9).

SEQ ID 8:
5'CACTCGAGCTAGTGGTGGTGGTGGTGGGAACCAAGGACTCTGTCCAAGTGTAC 3'
XhoI TER H H H H H H S G L V R D L H V
SEQ ID 9:
5 10 15 20 25 30
| | | | | |
1 M A P A R A G F C P L L L L L L G L W V A E I P V S A K P
31 K G M T S S Q W F K I Q H M Q P S P Q A C N S A M K N I N K
61 H T K R C K D L N T F L H E P F S S V A A T C Q T P K I A C
91 K N G D K N C H Q S H G P V S L T M C K L T S G K Y P N C R
121 Y K E K R Q N K S Y V V A C K P P Q K K D S Q Q F H L V P V
151 H L D R V L G S H H H H H

### Cell transfection

Cos1 cells were cultured in DMEM containing 2mM L-glutamic acid and 10% Fetal Bovine Serum (FBS). Subconfluent cells were transfected with CsC1-purified expression constructs or control expression vector at concentration of 5 µg of DNA/10cm² of cells, using the DEAE/dextran transfection method. One day after transfection, cells were placed on serum-free medium (1.5 ml of DAEM + 2mM L-glutamic acid) and incubated for 48h, after which the conditioned medium was collected, centrifuged at low speed and stored at -70°C until use.

### Prokaryotic expression of rKRF

### Construction of prokaryotic expression construct

A prokaryotic expression construct was made by PCR-subcloning the mature protein-encoding region of clone HB3661 into the prokaryotic expression vector pGEMT by PCR, using primer 7767 (SEQ ID 10) and primer 7768 (SEQ ID 11). This yielded clone N° HB3781 (pGEMTALFmat, ICCG 3242) encoding the secreted part of KRF (SEQ ID 9). The KRF-encoding insert from this clone was isolated by cutting with the restriction enzymes PmeI and XbaI and subcloned into the prokaryotic expression vector pIGRHIASA (ICCG 2075), which had previously been cut with BbrPI and XbaI. This yielded the prokaryotic KRF expression contruct pIGRHISAhALF containing the KRF gene downstream of the P1 promoter and fused with a His6-encoding sequence at its N-terminal end. Be full sequence of the protein expressed by this plasmid is given in SEQ ID 12.

SEQ ID 10
5'   CAGTTTAAACCCAAGGGCATGACCTCATC 3'
        PmleI
SEQ ID 11:
5'   AGTCTAGAAACCTAAAGGACTCTG 3'
        XbaI
SEQ ID 12:
  1  MAHHHHHHKP KGMTSSQWFK IQHMQPSPQA CNSAMKNINK HTKRCKDLNT FLHEPFSSVA
 61  ATCQTPKIAC KNGDKNCHQS HGPVSLTMCK LTSGKYPNCR YKEKRQNKSY VVACKPPQKK
121  DSQQFHLVPV HLDRVL

### Expression of rKRF in E. coli

Plasmid pIGRHISAhALF was transfected into E. coli strain MC1061pACI. Six liter-cultures of the transfected strain were prepared.

### Purification and refolding

*E. coli* cells from 2 liters of culture were pelleted and stored at -20°C until use. After thawing, the cell pellet was extracted with a 10-fold excess of extraction buffer containing 6M Guanidinium-hydrochloride and 20 mM NaH₂PO₄.H0 and homogenized using a Polytron mixer. Sulfonation agent was then added to 40 mM Tris, 100 mM Na₂SO₃, 20 mM Na₂S₄0₆, 100 µM CuSO₄, pH 8.5 and the sample incubated at room temperature for 6h. The sample was then centrifuged, to the supernatant 1% Empigen and 20 mM imidazole was added and the pH was lowered to 7.2. KRF was purified on a Ni-IMAC column and eluted with an imidazole step gradient made up in 6 M guanidinium hydrochloride, 50 mM phosphate buffer pH 7.2. Fractions containing KRF (as determined by gel electrophoresis and Western blot using anti-His antiserum were pooled and dialysed to 4M Ureum, 20 mM HEPES pH 7.2 in a 3.5 kDa cut-off membrane. After dialysis, a total of 8 ml purified KRF was obtained with a protein concentration of 1 mg/ml, as determined using the micro-BCA method. At this stage, the protein was >90% pure and migrated as a 15 kDa band, as determined by SDS-PAGE followed by silver staining and anti-His western blotting. Since it is known that the members of the RNase A family are disulfide bridge-containing proteins, the purified KRF was further treated by in vitro refolding. Therefore, 2 mg KRF was incubated overnight in presence of 20 ml of heparin TSK 650 resin, which was and equilibrated in buffer A (4M ureum, 20 mM HEPES, O.1% CHAPS, Ox/red. glutathione (2/10mM), pH 8). The gel was then packed in a XK16/20 column and washed with 100 ml of buffer A' (2M ureum, 20 mM HEPES, 0.1% CHAPS, 150 mM NaCl, Ox./Red glutathione, pH 8) and incubated for 2 h at room temperature. Subsequently, the gel was washed with 200 ml of buffer A" (20 mM HEPES, 0.1% CHAPS, 150 mM NaCl, 1% ethylene glycol, 1 mM lysine, Ox./Red glutathione, pH 8) and incubated overnight at 16°C. KRF was then eluted from the gel using a 0-3M NaCl step gradient in a buffer containing 20 mM HEPES, 0.1 % CHAPS, pH 7.2. The protein peak eluting at 1M NaCl was collected and stored in aliquots at -70°C until use.

### Example 3: Determination of RNase activity of recombinant KRF

### RNase assay

An RNase assay was set up as described by Lee and Vallee (1989): 10 µl of prediluted control (RNase A, Sigma) or test sample was added to 90 µl of a reaction solution containing 30 mM HEPES pH 6.8, 30 mM NaCl, 0.01% BSA and 0.03% tRNA. The reaction solution was incubated for 2 h at 37°C, after which the samples were placed on ice and 100 µl of 6% precooled perchloric acid was added. After an additional 10 min on ice, the samples were centrifuged for 10 min at 12,000 g (4°C) and the supernatant was diluted 1:3 in 1x reaction buffer (30 mM HEPES pH 6.8, 30 mM NaCl, 0.01% BSA). Then the OD was measured at 260 nm (higher OD values indicate increased degradation of the tRNA, resulting in a higher concentration of non-perchloric acid-precipitable oligonucleotides in the supernatant).

### RNase activity of rKRF

Upon assaying crude conditioned medium of cos1 cells transfected with the pSVDE51KRF and pSVDE51KRFH6 constructs, both media were found to contain RNase activity (Fig. 1, Fig. 2). The medium containing the his6-fusion protein appeared slightly less active than that containing the natural protein, although this could be due to lower transfection efficiency or expression level. After purification by Ni-IMAC, the cos-expressed KRF-His6 fusion protein has a specific activity which is about 9-fold lower than that of bovine pancreatic RNase A (Fig. 2). For comparison, the specific RNase activity of angiogenin is about 5x10⁴-fold lower than that of RNase A (Fig. 3).

The purified and refolded prokaryotic KRF-His6 fusion protein also displayed high RNase activity. The specific activity of this protein is about a 5-fold lower than that of the cos-expressed protein KRF-His6 fusion protein, suggesting an overall refolding efficiency of about 20%.

### Example 4: Antiviral activity of KRF

KRF is being evaluated for antiviral activity towards HBV, HCV and HIV.

For HBV, a test system based on the HepG2.2.15 hepatoma cell line is being used. This cell line contains two head-to-tail copies of the HBV genome and is constitutively producing HBV particles (Sells et al., 1987). This integrated HBV DNA, similar to the nuclear episome that is formed in a regular replication cycle, is a template for the host RNA polymerase that will produce four sets of RNA. As the RNase acts specifically on viral RNA, it inhibits further HBV replication. The inhibition is measured by a decrease in intracellular viral RNA transcripts (quantitative PCR, Northern Blot; Zhu et al, 1997), a decrease in viral DNA (quantitative PCR, Southern Blot; Korba and Boyd, 1996) and/or inhibition of HBsAg (surface antigen) production via ELISA (proprietary system comparable with commercially available ELISA). A second system, involving transfection of the HBV genome pCMVHBV (Fallows et al., 1995) into a hepatoma cell line, is also being used. A third system involves simulating the *in vivo* context by starting from an infectious HBV inoculum (Bchini et al, 1990).

HCV replication is currently evaluated in a human B-cell (Daudi). As HCV is an RNA virus, KRF has great potential as a HCV inhibitor. Daudi cells are being infected in the presence of KRF and inhibition of replication is measured using quantitative PCR (Gretch et al., 1996).

Activity towards HIV is tested in a CPE/MTT system (Pauwels et al., 1987). MT-4 cells (a human lymphocyte cell line) show a cytopathic effect (CPE) following infection with HIV. The cells are infected with HIV in the presence of a dilution series of compound. After 5 days, the surviving cells are monitored via a tetrazolium-based colorimetric MTT procedure. This allows to calculate the EC₅₀ (concentration of compound that protects 50% of the virus-infected cells against the viral cytopathic effect)(Pauwels et al., 1988). In all the abovementioned assay systems, the cytotoxic concentration of KRF is evaluated via a chemoluminescent method (Cytolite, Packard) or colorimetrically via MTT (Pauwels et al., 1988).

### Example 5: Angiogenic activity of KRF

Several systems for demonstrating angiogenic activity are known in the art (Jain et al., 1997). In a first system, endothelial cells are seeded on top of or into collagen gels (Montesano et al, 1986; Kanzawa et al, 1993; Jimi et al., 1995). Addition of an angiogenic factor then causes the cells to invade the gel and to reorganize them to form capillaries. The assay is made quantitative, for instance, by measuring the total length of the capillaries formed.

Other assays are based on in vivo observations. For instance, one assay is based on application of an angiogenic factor onto the chorioallantois membrane of fertilized chicken eggs. This causes an increase in the formation and invasion of new blood vessels in the treated area (Nguyen et al, 1994; Wilting et al, 1991; Ausprunk et al., 1975). Another in vivo assay involves the implantation of a slow release pellet containing an angiogenic factor under the cornea of rabbit eyes. Although the cornea is normally avascular, application of an angiogenic factor results in the formation of blood vessels in this tissue. This assay is very sensitive, since evaluation of angiogenesis is not biased by the presence of a background of blood vessels (Gaudric et al., 1992; Grant et al., 1993).

### Example 6: Wound repair stimulatory activity of KRF

One of the early events in the repair process after wounding is the formation of granulation tissue. This tissue consists of a loose provisional matrix of collagen I and III, filled with a dense capillary network. Angiogenesis constitutes a key process in the deposition of this granulation tissue (Pierce and Mustoe, 1995). Consequently, it is clear that a factor with angiogenic properties will be favourable to the healing process. Therefore, KRF is being evaluated for its tissue repair enhancing properties in a full thickness wound model in pigs (De Coninck et al., 1996). In this model system, square full-thickness wounds (2x2 cm) are created on the back of the animal. These wounds are then treated with KRF(different concentrations, ranging from 10ng to 100 µg per cm² of wound area) and with control substances (saline solution and hydrocolloid wound dressing). To optimize the delivery of KRF to the wound, it is formulated into a proprietary hydrogel wound dressing, which is in turn covered with a polyurethane dressing. At appropriate time points after wounding, the wounds are inspected and biopsy samples are taken. Parameters which are being evaluated include percentage of wound closure by epihelialisation and by contraction, degree of granulation tissue formation, degree of blood vessel formation, degree and organisation of collagen deposition and basement membrane formation.

### REFERENCES

- **Atherton, Shepard** (1989) Solid phase peptide synthesis. IRL Press, Oxford.
- **Ausprunk, D., Knighton, D. and Folkman, J.** (1975) Am J Pathol 79, 597-618. Vascularization of normal and neoplastic tissues grafted to the chick chorioallantois.
- **Bchini, R., Capel, F., Dauguet, C., Dubanchet, S. and Petit, M.-A.** (1990) J Virol 64, 3025-3032. *In vitro* infection of human hepatoma (HepG2) cells with hepatitis B virus.
- **Chomczynski, P and Sacchi, N** (1987) Anal Biochem 162, 156-159: Single-step method of RNA isolation by acid guanidinium thiocyanate-phenol-chloroform extraction
- **De Coninck, A., Draye, J.-P., Van Strubarq, A., Vanpée, E., Kaufman, L., Delaey, B., Verbeken, G. and Roseeuw, D.** (1996). J Dematol Sci 13, 202-211. Healing of full-thickness wounds in pigs: effects of occlusive and non-occlusive dressings associated with a gel vehicle.
- **Fallows, D.A. and Goff, S.P.** (1995) J Virol 69, 3067-3073. Mutations in the Σ sequences of human hepatitis B virus affect both RNA encapsidation and reverse transcription.
- **Fett, J., Strydom, D., Lobb, R., Alderman, E., Bethune, J., Riordan, J. and Vallee, B.** (1985) Biochemistry 24, 5480-5486. Isolation and chracterization of Angiogenin, an angiogenic protein from human carcinoma cells.
- **Gaudric, A., N'guyen, T., Moenner, M., Glacet-Bernard, A. and Barritault, D.** (1992) Ophtalmic Res 24, 181-188. Quantification of angiogenesis due to basic fibroblast growth factor in a modified rabbit corneal model.
- **Grant, M., Mames, R., Fitzgerald, C., Ellis, E. Aboufriekha, M. and Guy, J.** (1993) Diabetologia 36, 282-291. Insulin-like growth factor I acts as an angiogenic agent in rabbit cornea and retina: comparative studies with basic fibroblast growth factor.
- **Green, H, Kehinde O, Thomas J** (1979) Proc Natl Acad Sci USA 76, 5665-5668. Growth of cultured human epidermal cells into multiple epithelia suitable for grafting.
- **Gretch, D.R., Dela Rosa, C., Corey, L. and Carithers Jr., R.L.** (1996) Viral hepatitis reviews 2, 85-96. Assessment of hepatitis C viremia using molecu lar amplification techniques.
- **Griffiths, S., Adams, D. and Talbot, S.** (1997) Nature 390, 568. Ribonuclease inhibits Kaposi's sarcoma.
- **Houbenweyl** (1974) Methode der organischen chemie, vol. 15, I & II (ed. Wunch E). Thieme, Stuttgart.
- **Jain, R., Schlenger, K., Höckel, M. and Yuan, F.** (1997) Nature Med. 3, 1203-1208. Quantitative angiogenesis assays: progress and problems.
- **Jimi, S., Ito, K., Kohno, K, Onon, M., Kuwano, M. Itagaki, Y. and Ishikawa, H.** (1995) Biochem Biophys Res Commun 211, 476-483. Modulation by bovine angiogenin of tubular morphogenesis and expression of plasminogen activator in bovine endothelial cells.
- **Jinno, H., Ueda, M, Ozawa, S., Kikuchi, K., Kikeda, T., Enomoto, K. and Katajima, M.** (1996) Cancer Chemo & Pharmacol 38, 303-308. Epidermal growth factor receptor-dependent cytotoxic effect by an EGF-ribonuclease conjugate on human cancer cell lines - a trial for less immunogenic chimeric toxin.
- **Kanzawa, S., Endo, H. and Shioya, N.** (1993). Ann Plast Surg 30, 244-251. Improved in vitro angiogenesis model by collagen density reduction and the use of type III collagen.
- **King, T. and Vallee, B.** (1991) J Bone Joint Surg Br 73, 587-590. Neovascularisation
- **Korba, B.E. and Boyd, M.R.** (1996) Antimicrob Agents and Chemother 40, 1282-1284. Penciclovir is a selective inhibitor of hepatitis B virus replication in cultured human hepatoblastoma cells.
- **Lee, F. and Vallee, B.** (1989) Biochem Biophys Res Commun 161, 121-126. Characterization of ribonucleolytic activity of angiogenin towards tRNA.
- **Maniatis T, Fritsch E, Sambrook J** (1982) Molecular cloning: a laboratory manual. Cold Spring Harbor Laboratory Press, Cold Spring Harbor, NY.
- **Montesano, R., Vassalli, J.-D., Baird, A, Guillemin, R. and Orci, L.** (1986) Proc Natl Acad Sci USA 83, 7297-7301. Basic fibroblast growth factor incuces angiogenesis in vitro.
- **Newton, D., Walbridge, S., Mikulski, S., Ardelt, W., Shogen, K., Ackerman et al.** 1994 J Neuroscience 14, 538-544. Toxicity of an antitumor ribonuclease to Purkinje neurons.
- **Newton, D., Nicholls, P., Rybak, S. and Youle, R.** (1994b) J Biol Chem 269, 26739-26745. Expression and characterization of recombinant human eosinophil-derived neurotoxin and eosinophil-derived neurotoxin-anti-transferrin receptor sFv.
- **Newton, D., Pearson, J., Xue, Y., Smith, M., Fogler, W., Mikulski, S., Alvoerd, W., Kung, H., Longo, D. and Rybak, S.** (1996a). Int J Oncol 8, 1095-1104. Anti-tumor ribonuclease, combined with or conjugated to monoclonal antibody MRK16, overcomes multidrug resistance to vincristine in vitro and in vivo.
- **Newton, D., Xue, Y., Olson, K., Fett, J. and Rybak, S.** (1996) Biochem 35, 545-548. Angiogenin single-chain immunofusion - influence of peptide linkers and spacers between fusion protein domains.
- **Nguyen, M., Shing, Y. and Folkman, J.** (1994) Microvasc Res 47, 31-40. Quantitation of angiogenesis and antiangiogenesis in the chick embryo chorioallantoic membrane.
- **Pauwels, R., De Clercq, E., Desmyter, J., Balzarini, J., Goubau, P., Herdewijn, P., Vanderhaeghe, H. and Vandeputte, M.** (1987) J. Virol. Meth 16, 171-185. Sensitive and rapid assay on MT-4 cells for the detection of antiviral compounds against the AIDS virus.
- **Pauwels, R., Balzarini, J., Baba, M., Snoeck, R., Schols, D., Herdewijn, P., Desmyter, J. and De Clercq, E.** (1988) J. Virol. Meth. 20, 309-321. Rapid and automated tetrazolium-based colorimetric assay for the detection of anti-HIV compounds.
- **Pierce, G. and Mustoe, T.** (1995) Ann Rev Med 46, 467-481. Pharmacological enhancement of wound healing.
- **Rosenberg, H., Ackerman, S. and Tenen, D.** (1989) J Exp Med 170, 163-176. Human eosinophil cationic protein. Molecular cloning of a cytotoxin and helminthotoxim with ribonuclease activity.
- **Saxena, S., Rybak, S., Davey,, R., Youle, R. and Ackerman, E.** (1992) J Biol Chem 267, 21982-21986. Angiogenin is a cytotoxic, t-RNA-specific ribonuclease in the RNase A superfamily.
- **Saxena, S., Gravell, M., Wu, Y., Mikulski, S., Shogen, K., Ardelt, W. and Youle, R.** (1996) J Biol Chem, 271, 20783-20788. Inhibition of HIV-1 production and selective degardation of viral RNA by an amphibian ribonuclease.
- **Schein, C.** (1997) Nature Biotechnol 15, 529-536. From housekeeper to microsurgeon: the diagnostic and therapeutic potential of ribonucleases.
- **Sells, M.A., Chen, M.-L. and Acs, G.** (1987) Proc Natl Acad Sci USA 84, 1005-1009. Production of hepatitis B virus particles in HepG2 cells transfected with cloned hepatitis B virus cDNA.
- **Shapiro, R and Vallee, B.** (1989) Biochemistry 28, 7401-7408. Site-directed mutagenesis of histidine-13 and histidine-114 of human angiogenin. Alanine derivatives inhibit angiogenin-induced angiogenesis.
- **Shapiro, R. and Vallee, B.** (1992) Biochemistry 31, 12477-12485. Identification of functional arginines in human angiogenin by site-directed mutagenesis.
- **Wilting, J., Christ, B. and Bokeloh, M.** (1991) Anat Embryol 183, 259-271. A modified chorioallantoic membrane (CAM) assay for qualitative and quantitative study of growth factors.
- **Youle, R., Wu; Y., Mikulski, S., Shogen, K., Hamilton, R., Newton, D., D'Alessio, G. and Gravell, M.** (1994). Proc Natl Acad Sci USA 91, 6012-6016. RNase inhibition of human immunodeficiency virus infection of H9 cells.
- **Zewe, M., Rybak, S., Dubel, S., Coy, J, Welschof, M., Newton, D., Little, M.** (1997) Immunotechnology 3, 127-136. Cloning and cytotoxicity of a human pancreatic RNase immunofusion.
- **Zhu, Y.-L., Pai, S.B., Liu, S.-H, Grove, K.L., Jones, B.C.N.M., Simons, C., Zemlicka, J. and Cheng, Y.-C.** (1997) Antimicro. Agents Chemother 41, 1755-1760. Inhibition of replication of hepatitis B by cytallene *in vitro.*

## Claims

1. An isolated nucleic acid sequence encoding a polypeptide having the amino acid sequence of SEQ ID 5, or a derivative thereof having RNase, angiogenic, antiviral, cytotoxic or cytostatic activity, selected from the group consisting of:
a. the DNA sequence given by SEQ ID 4 or the complementary strand thereof,
b. DNA sequences which hybridize under stringent conditions to the protein coding regions of the DNA sequence delined in (a) or fragments thereof and
c. DNA sequences which, for the degeneracy of the genetic code, would hybridize to the DNA sequences defined in (a) and (b).

2. A vector comprising a nucleic acid according to claim 1.

3. A host cell comprising a vector according to claim 2.

4. A polypeptide having part or all of the amino acid sequence of SEQ ID 5 or any derivative thereof having RNase, angiogenic, antiviral, cytotoxic or cytostatic activity.

5. A pharmaceutical composition comprising a polypeptide according to claim 4, or any functionally equivalent variant or fragment thereof having RNase, angiogenic, antiviral, cytotoxic or cytostatic activity, and a pharmaceutically acceptable carrier.

6. A method for promoting angiogenesis in a mammal comprising the step of administering to said mammal a pharmaceutical composition according to claim 5.

7. A method for promoting wound healing in a mammal comprising the step of administering to said mammal a pharmaceutical composition according to claim 5.

8. A method for lowering the amount of intracellular or extracellular RNA in a mammal comprising the step of administering to said mammal a pharmaceutical composition according to claim 5.

9. A pharmaceutical composition according to claim 5 for use as medicament to treat a microbial infection.

10. A pharmaceutical composition according to claim 9 wherein said microbial infection is a viral infection.

11. A pharmaceutical composition according to claim 10 wherein said viral infection is caused by a RNA virus.

12. A pharmaceutical composition according to claim 11 wherein said RNA virus belongs to a RNA virus family chosen from the group consisting of Reoviridae, Coronaviridae, Flaviviridae, Picornaviridae, Togaviridae, Paramyxoviruses, Rhabdoviridae, Bunyaviridae, Orthomyxoviridae or Retroviridae.

13. A pharmaceutical composition according to claim 5 for use as medicament to treat malignancies and/or hyperproliferative disorders.

14. A method for detecting a nucleic acid sequence according to claim 1 comprising:
- contacting said nucleic acid with a probe
- determining the complex formed between said nucleic acid and said probe.

15. A method for detecting a polypeptide according to claim 4 comprising:
- contacting said polypeptide with a ligand binding to said polypeptide
- determining the complex formed between said polypeptide and said ligand.

16. A ligand binding to a polypeptide according to claim 4.
